# DEMANDE DE BREVET EUROPEEN

(11) **EP 1 738 748 A1**
(43) Date de publication de la demande: **03.01.2007**
(21) Numéro de dépôt: 06300700.9
(22) Date de dépôt: 05.09.2003
(51) Int. Cl.: A61K 8/891, A61K 8/31, A61K 8/37, A61K 8/81, A61Q 1/06

(54) **Composition cosmétique comprenant une huile siliconée phénylée, une huile hydrocarbonée non volatile, un agent rhéologique et une phase particulaire**

(30) Priorité: 06.09.2002 FR 0211095 F
(62) Demande divisionnaire de: 03020174.3
(71) Demandeur: L'Oreal, 75008 Paris (FR)
(72) Inventeur: Blin, Xavier, 75015 Paris (FR); Ferrari, Véronique, 94700 Maisons-Alfort (FR)
(74) Mandataire: Chantraine, Sylvie Hélène

(57) **Abrégé**

La présente invention a pour objet une composition comprenant, dans un milieu physiologiquement acceptable, au moins une huile siliconée phénylée de haute viscosité, au moins une huile hydrocarbonée non volatile ayant une masse moléculaire supérieure à 500 g/Mol et/ou un indice de réfraction à 20°C supérieur à 1,440, au moins un agent rhéologique et une phase particulaire.

La composition possède de bonnes propriétés de tenue, de brillance et de confort.

## Description

La présente invention se rapporte à une composition cosmétique comprenant, dans un milieu physiologiquement acceptable, une huile siliconée phénylée de haute viscosité, une huile hydrocarbonée non volatile, un agent rhéologique et une phase particulaire. Cette composition est notamment une composition de maquillage ou de soin de la peau, aussi bien du visage que du corps humain, y compris du cuir chevelu, des lèvres ou des phanères des êtres humains, comme les cheveux, les cils, les sourcils ou les ongles. Elle possède des propriétés cosmétiques remarquables, en particulier de tenue, et confère au maquillage ou au soin des propriétés de brillance et/ou de confort.

La composition de l'invention peut en particulier se présenter sous forme d'un produit de maquillage des matières kératiniques (peau, lèvres, phanères) ayant éventuellement des propriétés de soin et/ou de traitement non thérapeutique. Elle constitue notamment un rouge à lèvres ou un brillant à lèvres, un fond de teint, une poudre libre ou compactée, un fard à joues ou à paupières, une base de maquillage, un produit anticernes, un produit pour tatouage, un mascara, un eye-liner, un vernis à ongles, un produit de bronzage artificiel de la peau, un produit de coloration ou de soin des cheveux.

L'utilisation de composés siliconés dans des compositions cosmétiques, notamment de maquillage, est connue des formulateurs. On peut citer par exemple le document EP-A-0407205 qui décrit une composition comprenant l'association d'une gomme de silicone et d'une huile siliconée, présentant de bonnes propriétés de tenue, d'étalement et de confort. En particulier, cette association confère aux compositions cosmétiques d'excellentes propriétés sensorielles, notamment un toucher non gras, des propriétés d'étalement et de glissant et permettent d'obtenir sur la peau un film particulièrement homogène.

Il est également connu d'utiliser ces composés siliconés en vue d'augmenter la tenue des compositions cosmétiques, en particulier de maquillage. Les problèmes de mauvaise tenue se caractérisent par une modification de la couleur (virage, palissement) généralement par suite d'une interaction avec le sébum et/ou la sueur sécrétés par la peau dans le cas de fond de teint et de fard ou d'une interaction avec la salive dans le cas des rouges à lèvres. Ceci oblige l'utilisateur à se remaquiller très souvent, ce qui peut constituer une perte de temps.

Cependant, ces composés siliconés peuvent parfois poser des problèmes de formulation, et notamment des difficultés de dispersion des pigments dans les compositions comprenant des milieux siliconés, ce qui entraîne un mauvais développement de la teinte de la composition ainsi qu'un aspect granuleux, qui éloigne le consommateur de ce type de produit, et qui n'est pas favorable à l'obtention d'une composition brillante.

Or il existe de nombreuses compositions cosmétiques pour lesquelles les propriétés de brillance du film déposé, après application sur les matières kératiniques (peau, lèvres, phanères) sont très importantes. On peut citer par exemple les rouges à lèvres, les fards à paupières, les vernis à ongles ou encore certains produits capillaires.

Pour l'amélioration de la brillance, il est connu des formulateurs d'utiliser des huiles ayant une viscosité et un indice de réfraction élevés telles que des polymères huileux comme les polybutènes ou certaines huiles végétales (huile de ricin par exemple). Cependant, ces composés ne permettent pas l'obtention d'un film de composition dont la tenue, en particulier de la brillance, persiste au cours de la journée.

Le demandeur a constaté de façon surprenante que l'utilisation de l'association d'une huile siliconée phénylée de haute viscosité, d'une huile hydrocarbonée non volatile, d'un agent rhéologique et d'une phase particulaire permet l'obtention d'une composition présentant de bonnes propriétés cosmétiques et sensorielles, notamment de tenue dans le temps, même après un repas, de brillance et de confort et qui est non irritante pour les matières kératiniques.

La présente invention a plus précisément pour objet une composition comprenant, dans un milieu physiologiquement acceptable, au moins une huile siliconée phénylée de haute viscosité, au moins une huile hydrocarbonée non volatile ayant une masse moléculaire supérieure à 500 g/Mol et/ou un indice de réfraction à 20°C supérieur à 1,440, au moins un agent rhéologique et une phase particulaire.

La présente invention a également pour objet l'utilisation de l'association d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440, d'au moins un agent rhéologique, dans une composition comprenant un milieu physiologiquement acceptable et une phase particulaire, ladite composition présentant des propriétés de tenue et/ou de brillance et/ou de confort.

L'invention a également pour objet l'utilisation de l'association d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440 et d'au moins un agent rhéologique dans une composition comprenant un milieu physiologiquement acceptable et une phase particulaire, comme agent pour conférer de la tenue et/ou de la brillance et/ou du confort à ladite composition.

L'invention a encore pour objet un procédé cosmétique pour conférer à un film de composition cosmétique des propriétés de tenue et/ou de brillance et/ou de confort, consistant à introduire dans ladite composition une quantité efficace d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440, d'au moins un agent rhéologique et d'une phase particulaire.

Par "physiologiquement acceptable", on entend non toxique et susceptible d'être appliqué sur la peau (y compris l'intérieur des paupières), les lèvres ou les phanères d'êtres humains.

Par « au moins » un composé, on entend un ou plusieurs composés.

Par "huile", on entend tout milieu non aqueux liquide à température ambiante (25°C) et pression atmosphérique (760mm de Hg).

Par composé "non-volatil", on entend un composé susceptible de rester sur la peau ou les lèvres pendant plusieurs heures. Un composé non-volatil a en particulier une pression de vapeur, à température ambiante et pression atmosphérique, non nulle, inférieure à 0,02 mm de Hg (2,66 Pa).

Par composé "volatil", on entend un composé susceptible de s'évaporer de la peau ou des lèvres, en moins d'une heure. Un composé volatil est notamment choisi parmi les composés ayant une pression de vapeur, à température ambiante et pression atmosphérique allant de 0,02 mm à 300mm de Hg (2,66 Pa à 40 000 Pa) et mieux allant de 0,1 à 90 mm de Hg (13 Pa à 12 000 Pa).

Par huile siliconée phénylée "de haute viscosité", on entend une huile ayant une viscosité au moins égale à 500 cSt à 25°C.
Avantageusement, l'huile siliconée phénylée de haute viscosité présente une viscosité à 25 °C allant par exemple de 500 à 10 000 cSt, de préférence de 600 à 5000 cSt, et mieux de 600 à 3000 cSt.

De façon avantageuse, la composition selon l'invention comprend en outre une huile siliconée phénylée de basse viscosité qui présente une viscosité inférieure à 500 cSt à 25°C.

De préférence, l'huile siliconée phénylée de basse viscosité présente une viscosité à 25°C allant par exemple de 5 à 499 cSt, de préférence de 5 à 300 cSt, et mieux de 5 à 100 cSt.

L'huile siliconée phénylée de haute viscosité et l'huile siliconée phénylée de basse viscosité (si présente) peu(ven)t être par exemple une phényl trimethicone, une phényl dimethicone, une phényl triméthylsiloxy diphénylsiloxane, une diphényl diméthicone, une diphényl méthyldiphényl trisiloxane, ou un mélange de différentes huiles siliconées phénylées, et en particulier peu(ven)t répondre à la formule (A) suivante : 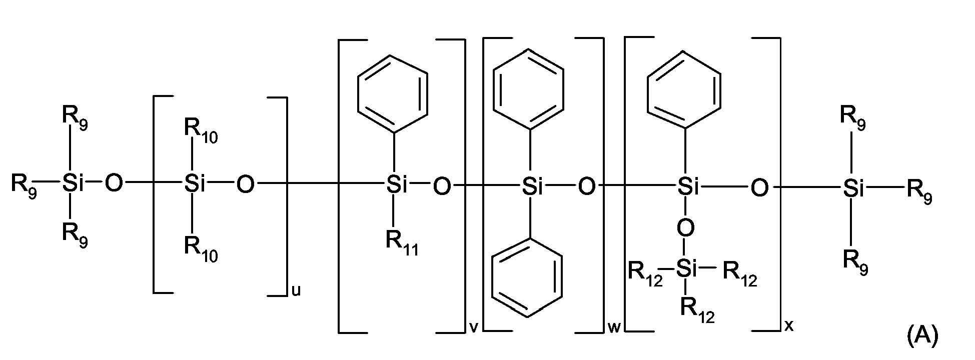 dans laquelle :
- R₉ et R₁₂ sont chacun indépendamment un radical alkyle en C₁-C₃₀, un radical aryle ou un radical aralkyle,
- R₁₀ et R₁₁ sont chacun indépendamment un radical alkyle en C₁-C₃₀ ou un radical aralkyle,
- u, v, w et x sont chacun indépendamment des nombres entiers allant de 0 à 900,
sous réserve que la somme v+w+x soit différente de 0 et que la somme u+v+w+x va de 1 à 900 ; en particulier, u+v+w+x va de 1 à 800.
De préférence, v est égal à 0.

De préférence, l'huile siliconée phénylée de basse viscosité satisfait à la formule (A) avec la somme u+v+w+x allant de 1 à 150, et mieux de 1 à 100, voire de 1 à 50 et l'huile siliconée phénylée de haute viscosité satisfait à la formule (A) avec la somme u+v+w+x allant de 151 à 900, mieux de 160 à 800, voire de 160 à 500.

En particulier, l'huile siliconée phénylée de basse viscosité satisfait à la formule (B) suivante : 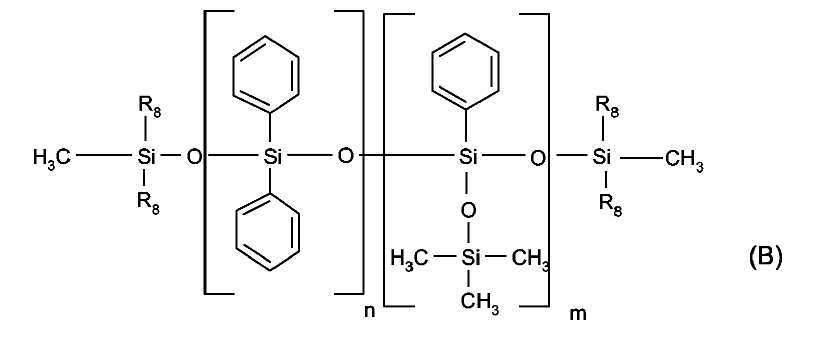 dans laquelle :
- R₈ est un radical alkyle en C₁-C₃₀, un radical aryle, ou un radical aralkyle,
- n est un nombre entier allant de 0 à 100, et mieux, inférieur à 100,
- m est un nombre entier allant de 0 à 100, et mieux, inférieur à 100,
sous réserve que la somme m+n va de 1 à 100 , et mieux, est inférieure à 100.

Parmi les huiles siliconées phénylées de haute viscosité utilisables dans l'invention, on peut citer les huiles 15 M 30 de PCR (500 cSt) ou la Belsil PDM 1000 (1000 cSt) de Wacker. Les valeurs entre parenthèses représentent les viscosités à 25°C.

Parmi les huiles siliconées phénylées de basse viscosité utilisables dans l'invention, on peut citer les huiles DC556 (22,5 cSt), SF558 (10-20 cSt) de Dow Corning, l'huile Abil AV8853 (4-6 cSt) de Goldschmidt, l'huile Silbione 70 633 V 30 (28 cSt) de Rhône Poulenc, les huiles 15 M 40 (50 à 100 cSt), 15 M 50 (20 à 25 cSt) de PCR, les huiles SF 1550 (25 cSt), PK 20 (20 cSt) de Bayer, les huiles Belsil PDM 200 (200 cSt) et Belsil PDM 20 (20cSt) de Wacker, les huiles KF 53 (175 cSt), KF 54 (400 cSt) et KF 56 (14 cSt) de Shin-Etsu.

L'huile siliconée phénylée de haute viscosité peut représenter de 5 à 99% du poids total de la composition, de préférence de 7,5 à 80%, mieux de 10 à 60% et encore mieux de 20 à 50%.

L'huile siliconée phénylée de basse viscosité (si présente) peut représenter de 5 à 99% du poids total de la composition, de préférence de 7,5 à 80%, mieux de 10 à 60% et encore mieux de 10 à 40%.

Le rapport en poids entre l'huile siliconée phénylée de basse viscosité et l'huile siliconée phénylée de haute viscosité peut aller par exemple de 1/10 à 10/1, de préférence de 2/10 à 10/2, mieux de 3/10 à 10/5. De façon préférentielle, ce rapport en poids est égal à 1/3.

La composition selon l'invention comprend également au moins une huile hydrocarbonée non volatile ayant une masse moléculaire supérieure à 500 g/Mol, de préférence supérieure à 600g/Mol, et mieux, supérieure à 650 g/Mol, mais ne dépassant pas 15000g/Mol et/ou un indice de réfraction supérieur à 1,440 à 20°C (l'indice de réfraction étant mesuré au réfractomètre), avantageusement supérieur à 1,450, et mieux, supérieur à 1,460.
Par composé "hydrocarboné", on entend un composé comportant principalement des atomes de carbone et d'hydrogène et éventuellement une ou plusieurs fonctions choisies parmi les fonctions hydroxyle, ester, éther, carboxylique. Ces composés sont en particulier exempts de groupements -Si-O-.

Cette huile hydrocarbonée non volatile peut être choisie parmi :
- les polymères lipophiles tels que :
   - les polybutylènes tels que l'INDOPOL H-100 (de masse molaire ou MM=965 g/mol), l'INDOPOL H-300 (MM=1340 g/mol), l'INDOPOL H-1500 (MM=2160g/mol) commercialisés ou fabriqués par la société AMOCO,
   - les polyisobutylènes hydrogénés tels que le PANALANE H-300 E commercialisé ou fabriqué par la société AMOCO (M =1340 g/mol, indice de réfraction de 1,498), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
   - Les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
- les esters tels que
   - les esters d'acides gras linéaires ayant un nombre total de carbone allant de 30 à 70 comme le tétrapélargonate de pentaérythrityle (MM=697,05 g/mol),
   - les esters hydroxylés tels que le malate de diisostéaryle (MM= 639 g/mol, indice de réfraction de 1,462),
   - les esters aromatiques tels que le tridecyl trimellitate (MM=757,19 g/mol),
   - les esters d'alcool gras ou d'acides gras ramifiés en C24-C28 tels que ceux décrits dans la demande EP-A-0 955 039, et notamment le citrate de triisocétyle (MM= 865 g/mol), le tétraisononanoate de pentaérythrityle (MM=697,05g/mol), le triisostéarate de glycéryle (MM=891,51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
   - les huiles d'origine végétale telles que l'huile de sésame (820,6 g/mol),
   - et leurs mélanges.

De préférence, l'huile hydrocarbonée non volatile est choisie parmi les esters d'acides gras linéaires ayant un nombre total de carbone allant de 30 à 70, les esters hydroxylés, les esters aromatiques, les esters d'alcool gras ou d'acides gras ramifiés en C24-C28 et leurs mélanges.

L'huile hydrocarbonée non volatile peut représenter de 5 à 99 %, de préférence de10 à 60 % et mieux de 15 à 50 % du poids total de la composition.

La composition selon l'invention contient au moins un agent rhéologique structurant de son milieu physiologiquement acceptable.

Cet agent rhéologique est capable d'épaissir et/ou gélifier la composition. Il peut être présent en une quantité efficace pour augmenter la viscosité de la composition, notamment jusqu'à l'obtention d'un gel solide, à savoir un produit ne s'écoulant pas sous son propre poids. Il est ainsi possible d'obtenir un stick.
Cet agent rhéologique est avantageusement choisi parmi les cires, les composés gras pâteux à température ambiante (25°C), les gélifiants lipophiles et leurs mélanges.

L'agent rhéologique peut représenter de 0,1 à 65%, de préférence de 1 à 50%, mieux de 3 à 40% et encore mieux de 5 à 30% du poids total de la composition.

Par "cire" au sens de la présente invention, on entend un composé gras lipophile, solide à température ambiante (25°C), à changement d'état solide/liquide réversible, ayant une température de fusion supérieure à 30°C, et mieux supérieure à 45°C, pouvant aller jusqu'à 150° C, une dureté supérieure à 0,5 Mpa à température ambiante, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Comme cires utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristallines, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ou encore le tétrastéarate de di-(triméthylol-1,1,1 propane) fabriqué ou commercialisé par la société Hétérène sous la dénomination HEST 2T- 4S et leurs mélanges.

De préférence, l'agent rhéologique comprend un mélange de cire microcristalline et de cire siliconée, telle que la cire alkyldimethicone ayant une chaîne alkyle en C30-C45.

La ou les cires peuvent être présentes en une quantité allant de 0,1 à 50% en poids par rapport au poids total de la composition, mieux de 1 à 30% et mieux de 3 à 25%.

L'agent rhéologique peut aussi être un composé gras pâteux à température ambiante (25°C). Par "composé gras pâteux", on entend un corps gras ayant une dureté, mesurée à température ambiante, allant de 0,001 à 0,5 MPa, de préférence allant de 0,005 à 0,4 MPa. Un pâteux a en outre un point de fusion compris entre 20 et 60°C, de préférence allant de 25 à 45°C.
Un composé pâteux est un produit visqueux comprenant une fraction solide et une fraction liquide.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées, le lanolate de magnésium ou les lanolines oxypropylènées et leurs mélanges. On peut également utiliser des esters d'acides gras ou d'alcools gras, notamment ceux ayant de 20 à 65 atomes de carbone (point de fusion de l'ordre de 20 à 35°C) comme le citrate de tri-isostéaryle, le propionate d'arachidyle, le polylaurate de vinyle ; les esters du cholestérol ; les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le « THIXINR » de Rheox ou encore le mélange de triglycérides d'acides laurique, myristique, palmitique et stéarique fabriqué ou commercialisé sous la référence Softisan 100 par la société Sasol.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le ou les corps gras pâteux peuvent être présents à raison de 0,1 à 60% en poids, par rapport au poids total de la composition, de préférence à raison de 1-45% en poids et encore plus préférentiellement à raison de 2-30% en poids, dans la composition, s'ils sont présents.

Le gélifiant lipophile peut être organique ou minéral, polymérique ou moléculaire. Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium ; la silice pyrogénée éventuellement traitée hydrophobe en surface dont la taille des particules est inférieure à 1µm. Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les copolymères d'un diacide en C₃₆ condensé sur l'éthylène diamine de masse moléculaire moyenne en poids d'environ 6000 tels que les composés commercialisés par la société Arizona Chemical sous les noms Uniclear 80 et Uniclear 100, les gommes notamment siliconées comme les PDMS ayant une viscosité > 100 000 centistokes, les galactommananes comportant de un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et leurs mélanges.

Comme gélifiants lipophiles préférés, on utilise des gélifiants organiques moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.
Par "phase grasse liquide", on entend, au sens de l'invention, une phase grasse liquide à température ambiante (25° C) et pression atmosphérique (760 mm de Hg, soit 105 Pa), composée d'un ou plusieurs corps gras liquides température ambiante, appelés aussi huiles, généralement compatibles entre eux. En particulier, cette phase grasse liquide est constituée par les huiles hydrocarbonées non volatiles décrites précédemment.

Le réseau supra-moléculaire peut résulter de la formation d'un réseau de fibrilles (dues aux empilements ou agrégations de molécules d'organogélateur), immobilisant les molécules de la phase grasse liquide.
L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.
Les interactions physiques sont diverses mais excluent la co-cristallisation. Ces interactions physiques sont en particulier des interactions du type interactions hydrogènes auto-complémentaires, interactions π entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogène et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en C₈ à C₂₂ tels que le dibutylamide de l'acide N-Lauroyl-L-glutamique, fabriqué ou commercialisé par la société Ajinomoto sous la dénomination GP-1 et leurs mélanges.

Le gélifiant lipophile peut représenter de 0,1 à 50 % en poids, de préférence de 1 à 30 % en poids, et mieux de 2 à 20 % en poids par rapport au poids total de la composition.

La phase particulaire présente dans la composition selon l'invention comprend des pigments et/ou des nacres et/ou des charges habituellement utilisés dans les compositions cosmétiques. La phase particulaire est généralement présente à raison de 0,01 à 60%, de préférence 10 à 5 à 25% en poids par rapport au poids total de la composition.

Selon l'invention, l'agent rhéologique est distinct de la phase particulaire et en particulier de la ou des charges éventuellement présents dans la phase particulaire.

Par pigments, il faut comprendre des particules blanches ou colorées, minérales ou organiques, insolubles dans les corps gras tels que les huiles, destinées à colorer et/ou opacifier la composition. Par charges, il faut comprendre des particules incolores ou blanches, minérales ou de synthèse, lamellaires ou non lamellaires. Par nacres ou pigments nacrés, il faut comprendre des particules irisées, notamment produites par certains mollusques dans leur coquille ou bien synthétisées.

Les pigments peuvent être blancs ou colorés, minéraux et/ou organiques, enrobés ou non, sphériques ou oblongues. On peut citer, parmi les pigments minéraux, le dioxyde de titane, éventuellement traité en surface, les oxydes de zirconium ou de cérium, ainsi que les oxydes de zinc, de fer (noir, jaune, brun ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium ou encore les laques à base de carmin de cochenille. Les pigments peuvent être présents dans la composition à raison de 0,05 à 40% du poids de la composition finale, et de préférence à raison de 2 à 20 % pour une composition non pulvérulente.

Les nacres ou pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. On peut aussi utiliser des pigments à propriétés goniochromatiques, notamment à cristaux liquides ou multicouches. Les nacres peuvent être présentes dans la composition à raison de 0,01 à 20 % du poids total de la composition, de préférence à un taux de l'ordre de 1 à 15 %.

Les charges peuvent être présentes à raison de 0,01 à 35 % (si présentes) du poids total de la composition, de préférence 0,5 à 15 %. On peut notamment citer le talc, le mica, le kaolin, la lauroyl lysine, les poudres de polyamide telles que le Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl® de Toshiba, par exemple).

De préférence, la composition de l'invention contient peu ou pas d'huiles volatiles et notamment moins de 10 % par rapport au poids total de la composition, de préférence moins de 5 % et mieux moins de 2 % et avantageusement est exempte d'huile volatile.

La composition selon l'invention peut comprendre en outre au moins un composé non aqueux additionnel différent de l'huile siliconée phénylée et de l'huile hydrocarbonée non volatile de masse moléculaire supérieur à 500 g/mol choisi parmi les huiles, des gommes, des résines, des polymères lipophiles et leurs mélanges.

Les gommes utilisables dans l'invention se présentent généralement sous forme solubilisée dans une huile et les résines peuvent être liquides ou solides à température ambiante.

La nature et la quantité des gommes et des résines sont fonction des propriétés mécaniques et des textures recherchées.

Les huiles additionnelles peuvent être des huiles hydrocarbonées et/ou siliconées et/ou fluorées. Ces huiles peuvent être d'origine animale, végétale, minérale ou synthétique. A titre d'exemple d'huile additionnelle utilisable dans l'invention, on peut citer :
- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec 10 ≤ R₁ + R₂ ≤ 41 comme par exemple, , le myristate d'isopropyle, le palmitate d'éthyl 2-hexyle, le stéarate d'octyl 2-dodécyle, l'érucate d'octyl 2-dodécyle ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les acides gras supérieurs en C₈-C₂₆ tels que l'acide oléique, l'acide linoléique, l'acide linolénique ou l'acide isostéarique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle ou alcoxy, pendant ou en bout de chaîne siliconée, groupements ayant de 2 à 24 atomes de carbone ;
- et leurs mélanges.

La composition de l'invention peut comprendre, en outre, au moins un additif usuellement utilisé dans le domaine concerné, tel que de l'eau, des colorants, des arômes, des parfums, des antioxydants, des conservateurs, des neutralisants, des gélifiants de phase aqueuse, des dispersants, des actifs cosmétiques, et leurs mélanges. Cet additif, à l'exception de l'eau qui peut représenter de 0,01 à 80 % et par exemple de 1 à 70 et mieux de 1 à 60 % du poids total de la composition, peut être présent dans la composition à raison de 0,0005 à 20% du poids total de la composition et mieux de 0,001 à 10%.

Par « actif cosmétique », on entend un composé lipophile ou hydrophile apportant un bénéfice aux matières kératiniques et plus spécialement à la peau et aux lèvres.

Comme actif cosmétique utilisable dans l'invention, on peut citer les vitamines A, E, C, B₃, F, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

Les applications de la composition selon l'invention sont multiples et concernent l'ensemble des produits cosmétiques colorés ou non et plus particulièrement les produits de maquillage des lèvres tels que les rouges à lèvres ou les brillants à lèvres ou encore les crayons à lèvres.

Dans un mode de réalisation particulier de l'invention, la composition selon l'invention peut se présenter sous forme d'un produit coulé, et par exemple sous la forme d'un stick ou bâton, ou sous la forme de coupelle utilisable par contact direct ou à l'éponge ou encore dans une bouillotte. En particulier, elle se présente sous forme solide et trouvent alors une application en tant que fond de teint coulé, fard à joues ou à paupières coulé, rouge à lèvres, base ou baume de soin pour les lèvres, produit anti-cernes. Elle peut aussi se présenter sous forme un fond de teint ou un rouge à lèvres fluides, un brillant à lèvres (gloss en terminologie anglo-saxonne), un produit solaire ou de coloration de la peau.

La composition de l'invention peut être anhydre et contenir moins de 5 % d'eau ajoutée par rapport au poids total de la composition. Elle peut alors se présenter notamment sous forme de gel huileux, de liquide huileux, de pâte ou de stick anhydre contenant notamment une dispersion vésiculaire de lipides ioniques et/ou non ioniques.
Elle peut aussi se présenter sous forme d'une émulsion simple ou multiple à phase continue huileuse ou aqueuse, de dispersion huileuse dans une phase aqueuse grâce à des vésicules contenant des lipides ioniques et/ou non ioniques. Ces formes galéniques sont préparées selon les méthodes usuelles des domaines considérés.
La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous la forme d'un produit de maquillage des lèvres tel qu'un rouge à lèvres ou un brillant à lèvres.

Un produit de maquillage des lèvres se présente avantageusement sous forme anhydre.

Bien entendu la composition de l'invention doit être physiologiquement acceptable, à savoir non toxique et susceptible d'être appliquée sur la peau (y compris l'intérieur des paupières), les lèvres ou les phanères d'êtres humains. Elle est en particulier cosmétiquement acceptable, c'est à dire agréable de goût, de toucher, d'aspect et/ou d'odeur, applicable plusieurs fois par jour pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

### Exemples 1 à 4 : Essais comparatifs - Sticks de Rouges à Lèvres

Les compositions figurant dans le tableau (1) ci-après ont été réalisées :
- la composition de l'exemple 1 comprend une huile siliconée, le cyclopentasiloxane,
- la composition de l'exemple 2 comprend une huile hydrocarbonée volatile, l'isododécane de masse moléculaire 170 g/Mol,
- la composition de l'exemple 3 comprend une huile hydrocarbonée non volatile, l'isononanoate d'isononyle ayant une masse moléculaire égale à 284 g/Mol et un indice de réfraction de 1,436,
- la composition de l'exemple 4 selon l'invention comprend du diisostéaryl malate ayant une masse moléculaire égale à 639 g/Mol et un indice de réfraction de 1,462.

**Tableau (1)**

| Phase | | **Exemple 1 (comparatif)** | **Exemple 2 (comparatif)** | **Exemple 3 (comparatif)** | **Exemple 4 (invention)** |
|---|---|---|---|---|---|
| **A** | Cyclopentasiloxane | 30 | | | |
| | Isododécane | | 30 | | |
| | Isononanoate d'isononyle | | | 30 | |
| | Malate de di-isostéaryle | | | | 30 |
| | Phényltrimethyltrisiloxane (20 cSt) fabriquée ou commercialisée par la société Dow Corning sous la référence DC 556 | 18 | 18 | 18 | 18 |
| | Phényltrimethyltrisiloxane (1000 cSt) fabriquée ou commercialisée par la société Wacker sous la référence Belsil PDM 1000 | Qsp 100 | Qsp 100 | Qsp 100 | Qsp 100 |
| **B** | Cire microcristalline | 10 | 10 | 10 | 10 |
| | Alkyl diméthicone en C₃₀-C₄₅ | 2,5 | 2,5 | 2,5 | 2,5 |
| | Mélange de triglycérides d'acides laurique, myristique, palmitique et stéarique (50/20/10/10) fabriqué ou commercialisé sous la référence Softisan 100 par la société Sasol. | 10 | 10 | 10 | 10 |
| **C** | Red 7 | 0,26 | 0,26 | 0,26 | 0,26 |
| | Red 21 | 0,06 | 0,06 | 0,06 | 0,06 |
| | Oxyde de fer noir | 0,09 | 0,09 | 0,09 | 0,09 |
| | Oxyde de fer brun | 2,1 | 2,1 | 2,1 | 2,1 |
| | Mica oxyde de titane | 1,8 | 1,8 | 1,8 | 1,8 |

### Mode opératoire

Les pigments (phase C) sont broyés dans le malate de diisostéaryle de la phase A, puis le broyat est mélangé à la phase (B) (cires et pâteux) et au restant de la phase A. Le mélange est chauffé dans un poêlon double enveloppe pendant au moins 30 minutes après fonte totale des cires.
La pâte obtenue est coulée dans un moule approprié pour stick qui est porté à 40-42 °C et qui est ensuite placé à -18°C pendant une demi-heure. Puis les sticks sont démoulés.

### Evaluation cosmétique

Les 4 sticks de rouge à lèvres ont été évalués par 5 personnes qualifiées selon différents critères.

Les sticks des exemples 2 et 3 ont été jugés comme ayant de mauvaises propriétés de dépôt car de consistance trop molle ; le stick de l'exemple 1 a été jugé comme déposant bien sur les lèvres mais présentant une perte de brillance dans le temps.
Le stick de l'exemple 4 selon l'invention a été jugé comme déposant bien, le film de composition a été jugé homogène et brillant.

### Exemple 5 : Stick de Rouge à lèvres

| Phase | | |
|---|---|---|
| A | Malate de di-isostéaryle | qsp 100 |
| | Phényltrimethyltrisiloxane (20 cSt) fabriquée ou commercialisée par la société Dow Corning sous la référence DC 556 | 18 |
| | Phényltrimethyltrisiloxane (1000 cSt) fabriquée ou commercialisée par la société Wacker sous la référence Belsil PDM 1000 | 27 |
| B | Cire microcristalline | 10 |
| | Alkyl diméthicone en C30-C45 | 2,5 |
| | Mélange de triglycérides d'acides laurique, myristique, palmitique et stéarique (50/20/10/10) fabriqué ou commercialisé sous la référence Softisan 100 par la société Sasol. | 10 |
| C | Red 7 | 0,26 |
| | Red 21 | 0,06 |
| | Oxyde de fer noir | 0,09 |
| | Oxyde de fer brun | 2,1 |
| | Mica oxyde de titane | 1,8 |

Le mode opératoire est le même que celui des exemples 1 à 4.

### Evaluation cosmétique :

La tenue de cette composition a été évaluée à l'aide de méthodes instrumentales et sensorielles sur un panel de 12 personnes qualifiées qui ont appliqué le stick de rouge à lèvres.

L'évaluation de la tenue se déroule de la façon suivante :
- dans un premier temps, une évaluation de la tenue "sensorielle" globale est réalisée une heure après l'application de la formule sur les lèvres.
- dans un second temps, une tenue "instrumentale" est évaluée après une série d'épreuves consistant à faire deux « bises » sur un mouchoir en papier, boire une boisson chaude puis une boisson froide et manger 4 bouchées d'un sandwich et d'une pomme.

La tenue sensorielle est évaluée sur une échelle allant de 1 à 10 : 1 correspond à une formule qui ne tient pas du tout et 10 à une formule qui tient très bien.
La tenue instrumentale est évaluée sur une échelle allant de 1 à 100 : 1 correspond à une formule qui ne tient pas du tout et 100 à une formule qui tient très bien.

La brillance et le confort ont aussi été évalués par ces 12 personnes :
- la brillance a été évaluée juste après l'application de la formule puis au bout d'une heure (la brillance instrumentale est évaluée sur une échelle allant de 1 à 200. 1 correspond à une formule qui n'est pas du tout brillante et 200 à une formule qui est très brillante.
   La brillance instrumentale est mesurée à l'aide d'un brillancemètre, de manière conventionnelle, par la méthode suivante.
   Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRI-GLOSS.
- le confort a été évalué au bout d'une heure.

### Résultats

| | | **Tenue** | **Brillance** | **Confort** |
|---|---|---|---|---|
| **Evaluation sensorielle** | A l'application | | 6,3 | |
| | A 1 heure | 6,4 | 4,8 | 7,4 |
| **Evaluation instrumentale** | A l'application | | 171 | |
| | A 1 heure | 82 | 138 | |
| | Après épreuves | 54 | | |

La composition présente de bonnes propriétés cométiques, notamment de brillance et de confort, et sa tenue dans le temps est très satisfaisante.
L'application (facilité d'application et glissant) du film de composition a également été jugée comme satisfaisante.

## Revendications

1. Composition comprenant, dans un milieu physiologiquement acceptable, au moins une huile siliconée phénylée de haute viscosité, au moins une huile hydrocarbonée non volatile ayant une masse moléculaire supérieure à 500 g/Mol et/ou un indice de réfraction à 20°C supérieur à 1,440, au moins un agent rhéologique et une phase particulaire.

2. Composition selon la revendication précédente, **caractérisée en ce que** l'huile hydrocarbonée non volatile a une masse moléculaire supérieure à 600 g/Mol.

3. Composition selon l'une des revendications précédentes, **caractérisée en ce que** l'huile hydrocarbonée non volatile a un indice de réfraction à 20°C supérieur à 1,450.

4. Composition selon l'une des revendications précédentes, **caractérisée en ce que** l'huile hydrocarbonée non volatile est choisie parmi les polybutylènes, les polyisobutylènes hydrogénés, les polydécènes, les polydécènes hydrogénés, les esters d'acides gras linéaires ayant un nombre total de carbone allant de 30 à 70, les esters hydroxylés, les esters aromatiques, les esters d'alcool gras ou d'acides gras ramifiés en C24-C28, les huiles d'origine végétale et leurs mélanges.

5. Composition selon l'une des revendications précédentes, **caractérisée en ce que** l'huile hydrocarbonée non volatile est choisie parmi le tétrapélargonate de pentaérythrityle, le malate de diisostéaryle, le tridécyl trimellitate, le citrate de triisocétyle, le tétraisononanoate de pentaérythrityle, le triisostéarate de glycéryle, le tri décyl-2 tétradécanoate de glycéryle, le tétraisostéarate de pentaérythrityle et leurs mélanges.

6. Composition selon l'une des revendications précédentes, **caractérisée en ce que** l'huile hydrocarbonée non volatile représente de 5 à 99 %, de préférence de 10 à 60 % et mieux de 15 à 50 % du poids total de la composition.

7. Composition selon l'une des revendications précédentes, **caractérisée en ce que** l'huile siliconée phénylée de haute viscosité présente une viscosité à 25°C allant de 500 à 10 000 cSt.

8. Composition selon l'une des revendications précédentes, **caractérisée en ce que** l'huile siliconée phénylée de haute viscosité présente une viscosité à 25°C allant de 600 à 5000 cSt.

9. Composition selon l'une des revendications précédentes, **caractérisée en ce que** l'huile siliconée phénylée de haute viscosité présente une viscosité à 25°C allant de 600 à 3000 cSt.

10. Composition selon l'une des revendications précédentes, **caractérisée en ce que** l'huile siliconée phénylée de haute viscosité représente de 5 à 99% en poids de la composition.

11. Composition selon l'une des revendications précédentes, **caractérisée en ce qu'**elle contient en outre une huile siliconée phénylée de basse viscosité.

12. Composition selon la revendication précédente, **caractérisée en ce que** l'huile siliconée phénylée de basse viscosité présente une viscosité à 25°C allant de 5 à 499 cSt.

13. Composition selon la revendication 11 ou 12, **caractérisée en ce que** l'huile siliconée phénylée de basse viscosité présente une viscosité à 25°C allant de 5 à 300 cSt à 25°C.

14. Composition selon l'une des revendications 11 à 13, **caractérisée en ce que** l'huile siliconée phénylée de basse viscosité présente une viscosité à 25°C allant de 5 à 100 cSt à 25°C.

15. Composition selon l'une des revendications précédentes, **caractérisée en ce que** l'huile siliconée phénylée de haute viscosité et/ou l'huile siliconée phénylée de basse viscosité est choisie parmi les huiles de formule (A) suivante : 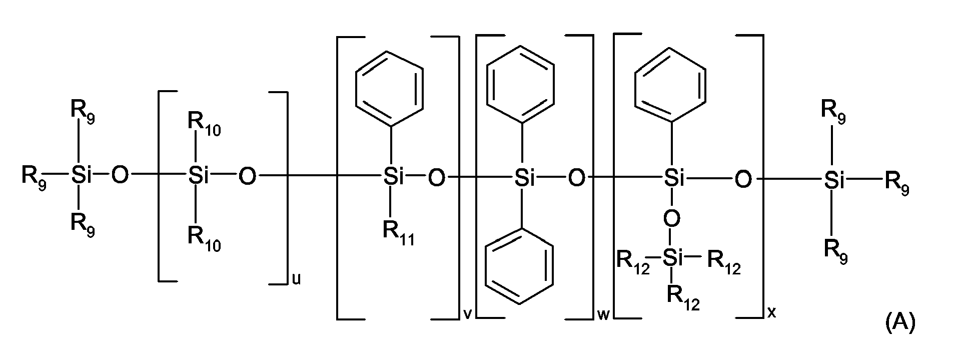 dans laquelle :
- R₉ et R₁₂ sont chacun indépendamment un radical alkyle en C₁-C₃₀, un radical aryle ou un radical aralkyle,
- R₁₀ et R₁₁ sont chacun indépendamment un radical alkyle en C₁-C₃₀ ou un radical aralkyle,
- u, v, w et x sont chacun indépendamment des nombres entiers allant de 0 à 900,
sous réserve que la somme v+w+x soit différente de 0 et que la somme u+v+w+x va de 1 à 900, en particulier, u+v+w+x va de 1 à 800.

16. Composition selon l'une des revendications 11 à 15, **caractérisée en ce que** l'huile siliconée phénylée de basse viscosité représente de 5 à 99% en poids de la composition.

17. Composition selon l'une des revendications 11 à 16, **caractérisée en ce que** le rapport en poids entre l'huile siliconée phénylée de basse viscosité et l'huile siliconée de haute viscosité va de 1/10 à 10/1.

18. Composition selon l'une des revendications 11 à 17, **caractérisée en ce que** le rapport en poids entre l'huile siliconée phénylée de basse viscosité et l'huile siliconée de haute viscosité va de 2/10 à 10/2.

19. Composition selon l'une des revendications 11 à 18, **caractérisée en ce que** le rapport en poids entre l'huile siliconée phénylée de basse viscosité et l'huile siliconée de haute viscosité va de 3/10 à 10/5.

20. Composition selon l'une des revendications précédentes, **caractérisée en ce que** l'agent rhéologique est choisi parmi les cires, les composés gras pâteux à température ambiante (25°C), les gélifiants lipophiles et leurs mélanges.

21. Composition selon l'une des revendications précédentes, **caractérisée en ce que** l'agent rhéologique représente de 0,1 à 65%, de préférence de 1 à 50%, mieux de 3 à 40% et encore mieux de 5 à 30% du poids total de la composition.

22. Composition selon la revendication 20 ou 21, **caractérisée en ce que** la cire est choisie parmi la cire d'abeilles, la cire de Carnauba, de Candelilla, d'Ouricoury, du Japon, de fibres de liège ou de canne à sucre, de riz, de Montan, la paraffine, les cires de lignite ou microcristallines, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ou encore le tétrastéarate de di-(triméthylol-1,1,1 propane) et leurs mélanges.

23. Composition selon la revendication 21 ou 22, **caractérisée en ce que** la cire représente de 0,1 à 50% en poids par rapport au poids total de la composition, mieux de 1 à 30% et mieux de 3 à 25%.

24. Composition selon la revendication 20, **caractérisée en ce que** le composé gras pâteux est choisi parmi les lanolines et les dérivés de lanoline, les esters d'acides gras ou d'alcools gras ayant de 20 à 65 atomes de carbone, les esters du cholestérol, les triglycérides d'origine végétale, les polyesters visqueux et leurs mélanges.

25. Composition selon la revendication précédente, **caractérisée en ce que** le composé gras pâteux représente de 0,1 à 60%, de préférence de 1 à 45% et mieux de 2 à 30% en poids par rapport au poids total de la composition

26. Composition selon la revendication 20, **caractérisée en ce que** le gélifiant lipophile est un organogélateur.

27. Composition selon la revendication précédente, **caractérisée en ce que** l'organogélateur est choisi parmi les amides d'acides tri-carboxyliques, les diamides ayant des chaînes hydrocarbonées contenant chacune de 1 à 22 atomes de carbone, lesdites chaînes étant non substituées ou substituées avec au moins un substituant choisi parmi les groupes ester, urée et fluoro, les amides de N-acylamino acides et leurs mélanges.

28. Composition selon l'une des revendications 26 ou 27, **caractérisée en ce que** l'organogélateur est choisi parmi les cyclohexanetricarboxamides, les diamides résultant de la réaction du diaminocyclohexane et d'un chlorure d'acide, les diamides résultant de l'action d'un N-acylamino acide avec des amines comportant de 1 à 22 atomes de carbone et leurs mélanges.

29. Composition selon l'une des revendications 26 à 28, **caractérisée en ce que** le gélifiant lipophile représente de 0,1 à 50%, mieux 1 à 30 %, et mieux de 2 à 20 % en poids par rapport au poids total de la composition.

30. Composition selon l'une des revendications précédentes, **caractérisée en ce que** la phase particulaire comprend des pigments et/ou des nacres et/ou des charges.

31. Composition selon l'une des revendications précédentes, **caractérisée en ce que** la phase particulaire est présente à raison de 0,01 à 60%, de préférence 5 à 25% en poids par rapport au poids total de la composition.

32. Composition selon l'une des revendications précédentes, **caractérisée en ce qu'**elle comprend un composé non aqueux additionnel.

33. Composition selon l'une des revendications précédentes, **caractérisée en ce qu'**elle se présente sous la forme d'un produit de maquillage et/ou de soin du visage ou du corps, des lèvres et/ou des phanères.

34. Composition selon l'une des revendications précédentes, **caractérisée en ce qu'**elle se présente sous la forme d'un produit de maquillage des lèvres.

35. Composition selon la revendication précédente, **caractérisée en ce qu'**elle se présente sous forme anhydre.

36. Utilisation de l'association d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440 et d'au moins un agent rhéologique, dans une composition comprenant un milieu physiologiquement acceptable et une phase particulaire, ladite composition présentant des propriétés de tenue et/ou de brillance et/ou de confort.

37. Utilisation de l'association d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440 et d'au moins un agent rhéologique dans une composition comprenant un milieu physiologiquement acceptable et une phase particulaire, comme agent pour conférer de la tenue et/ou de la brillance et/ou du confort à ladite composition.

38. Procédé cosmétique pour conférer à un film de composition cosmétique des propriétés de tenue et/ou de brillance et/ou de confort, consistant à introduire dans ladite composition une quantité efficace d'au moins une huile siliconée phénylée de haute viscosité, d'au moins une huile hydrocarbonée non volatile ayant une masse moléculaire supérieure à 500 g/Mol et/ou un indice de réfraction à 20°C supérieur à 1,440, d'au moins un agent rhéologique et d'une phase particulaire.
